# EUROPEAN PATENT SPECIFICATION

(11) **EP 3 685 800 B1**
(45) Date of publication and mention of the grant of the patent: **25.03.2026**
(21) Application number: 19382042.0
(22) Date of filing: 22.01.2019
(51) Int. Cl.: A61F 2/14

(54) **CORNEAL IMPLANT**
HORNHAUTIMPLANTAT
IMPLANT CORNÉEN

(43) Date of publication of application: 29.07.2020
(73) Proprietor: AJL Ophthalmic, S.A., 01510 Miñano (Araba/Alava) (ES); Fundación de Investigación Oftalmológica Fernández -Vega, 33012 Oviedo (Asturias) (ES)
(72) Inventor: LARRA MATEOS, Eva, 01510 MIÑANO (ARABA/ÁLAVA) (ES); PEREZ GRACIA, Jesús, 01510 MIÑANO (ARABA/ÁLAVA) (ES); ALFONSO SÁNCHEZ, José, Fernando, 33429 Urb. La Fresneda-Siero (Asturias) (ES)
(74) Representative: Herrero & Asociados, S.L.

(56) References cited:
- WO-A1-01/95836
- WO-A1-2017/177145
- US-A1- 2006 100 704
- US-A1- 2006 235 513
- US-A1- 2010 069 915

## Description

The present invention relates to a corneal implant, particularly the implant is useful for a corneal transplant, the implant avoid the direct contact between the patient tissue and a donated cornea.

### BACKGROUND ART

Diseases affecting the cornea are a major cause of global vision loss. Some pathology may lead to corneal dysfunction necessitating corneal transplantation.

There are three basic types of corneal transplantations. Full thickness transplants, the anterior lamellar transplant, if we replace the epithelium and the stroma, and the posterior lamellar transplant, if we replace the endothelium.

Complications of a corneal transplant can be significant and can include cornea graft rejection and eye infection.

Rejection of the donor tissue is the most serious complication after a corneal transplant.

The patent document US8388608 describes the developing of a new method for attaching the donor transplant cornea or artificial cornea without the use of sutures by means of a special implant. This method try to overcome the problems associated with the sutures based on precise cuts in the cornea.

While full thickness corneal transplant have not changed much over the past century, lamellar corneal transplant techniques have evolved rapidly.

In some cases when there was a bad prognosis for a full thickness transplant, the possibility of associating a prosthesis to the donor cornea with the intention of improving this prognosis was proposed.

One example is the Boston keratoprothesis. It was originally made of PMMA in a collar button design, to be implanted into a corneal graft carrier and then transplanted to the patients' cornea. The risks associated are; postoperative infections and again the main problem is the rejection of the donor tissues. Also ocular hypertension, and mechanical weakness of the cornea in case of trauma are problems arising from perforation of the cornea.

In connection with this techniques of lamellar transplant the patent EP1428470 describes an apparatus for determining and ablating a corneal tissue volume of a receiving cornea for corneal lamellar grafting transplantation.

The prognosis of corneal transplants with the introduction of these lamellar techniques has improved significantly, so today they are considered to be the first choice techniques as long as the criteria for their surgical indication are met.

US 2010/069915 teaches an implant with no disc as planar element and without the functionality set out in present claim 1.

Thus, from what is known in the art, it is derived that there is still of great interest to develop an implant transplant to improve some of the problems associated with the actual techniques.

### SUMMARY OF THE INVENTION

The cornea is the transparent layer forming the front of the eye. The tissues of the cornea are arranged in three basic layers: epithelium-bowman, stroma and descemet-endothelium.

The epithelium-bowman is the cornea's outermost layer. The second layer is the stroma, which is the thickest layer of the cornea. The descemet-endothelium is the innermost layer of the cornea.

The corneal transplantation is a widely practiced surgical procedure. There are two main types of cornea transplant: full thickness cornea and layer cornea transplant wherein not all the cornea is eliminated, called lamellar transplantation.

The present implant is useful in the transplant wherein not all the corneal's layer are eliminated. The implant of the present invention is a mechanical barrier between the donor's cornea and the host descemet-endothelium, i.e. the patient's descemet-endothelium.

When the two superficial layers of the cornea, epithelium and stroma are eliminated (as when it is carried out a previous lamellar transplant) a donor cornea replaced them, with its total thickness, that is, with its three layers, epithelium-bowman, stroma and descemet-endothelium. In the space formed between the donor cornea and the deep layer of the receiving cornea, the implant of the invention is inserted in order to keep both corneas separated.

Therefore, the present invention relates to an implant as defined in claim 1.

The implant of the invention shows many advantages, it is minimized the risk of the rejection because there is not a contact between the donor and the host tissues.

The method to implant the implant of the invention comprises the following steps:
a) eliminating the epithelium-bowman and stroma layers of the host;
b) positioning the implant defined above on the host's descemet-endothelium with the planar element by direct contact with the descemet-endothelium and the ring in a superior plane parallel to the planar element;
c) positioning the donor's cornea in the ring of the implant;
d) suturing the donor's and the host's corneas.

The donor's tissue and the patient (host) tissue are separated; there is a chamber between them originated from the implant. This chamber is similar to the anterior chamber of the ocular globe (we name it, pseudochamber).

In the method described above the patient cornea is not perforated. The perforation is one of the steps that cause the most serious problems in the cornea's transplantation.

After the placement of the implant in its correct position, over the descemet-endothelium and the positioning of the donor's cornea on top of it, on the ring, the donor's cornea is sutured with the peripheral corneal part of the patient, to ensure its union and favor a correct healing.

### BRIEF DESCRIPTION OF THE DRAWINGS

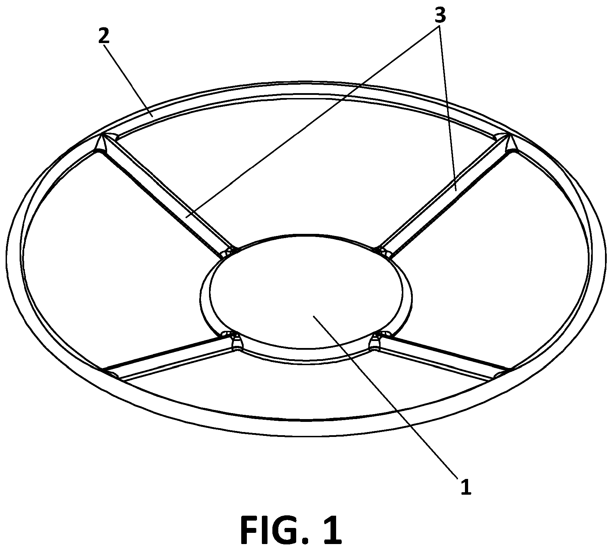

FIG. 1 shows a perspective view of the implant.

### DETAILED DESCRIPTION OF THE INVENTION

As mentioned above, an aspect of the present invention relates to an implant comprising:
a planar element in the centre of the implant, a ring in a superior plane parallel to the planar element, and a radial connecting element between the planar element and the ring.

The connecting element between the central planar element and the ring is situated in the radial direction, from the centre of the implant to the ring.

The planar element is a disc.

The term disc means a flat, circular element.

In a particular embodiment the diameter of the ring is greater than the diameter of the disc.

In a particular embodiment the diameter of the ring is comprised between 7.5 mm and 9.0 mm. More particularly the diameter of the ring is comprised between 8.0 mm and 8.5 mm.

In a particular embodiment the connecting element is at least a rib interconnecting the planar element and the ring in the superior plane. More particularl the at least a rib are four ribs separated to one another in azimuthal direction by an angle of 90°.

Particularly the implant is made of poly (methyl methacrylate) (PMMA).

The poly (methyl methacrylate) is a hard rigid, glassy but brittle material. Also it is an inert material usually used in intraocular lenses and hard contact lenses. PMMA has also been used in intraocular implants and as intrastromal corneal rings due to its mechanical and optical properties.

The Figure 1 shows a perspective of the implant. In this particularly embodiment the implant shows a central disc (1) in the centre of the implant, a ring (2) in a superior parallel plane and four ribs (3) connecting the two elements, disc (1) and ring (2).

## Claims

1. An implant comprising:
a planar element (1) in the centre of the implant, a ring (2) in a superior plane parallel to the planar element, and a radial connecting element (3) between the planar element and the ring wherein the planar element is a disc (1) wherein the implant is configured to perform the function of a mechanical barrier between the donor's cornea and the host descemet endothelium.

2. The implant according to claim 1 wherein the diameter of the ring (2) is greater than the diameter of the disc (1).

3. The implant according to claim 1 wherein diameter of the ring (2) is comprised between 7.5 mm and 9.0 mm.

4. The implant according to claim 1 wherein the diameter of the ring (2) is comprised between 8.0 mm and 8.5 mm.

5. The implant according to any of the claims 1 to 4 wherein the radial connecting element is at least a rib (3) interconnecting the planar element (1) and the ring (2) in the superior plane.

6. The implant according to claim 5 wherein the at least a rib are four ribs (3) separated to one another in azimuthal direction by an angle of 90°.

## Patentansprüche

1. Implantat, umfassend:
ein ebenes Element (1) in der Mitte des Implantats,
einen Ring (2) in einer oberen Ebene parallel zu dem ebenen Element, und
ein radiales Verbindungselement (3) zwischen dem ebenen Element und dem Ring, wobei
das ebene Element eine Scheibe (1) ist, wobei
das Implantat dazu eingerichtet ist, die Funktion einer mechanischen Barriere zwischen der Hornhaut des Spenders und dem Descemet-Endothel des Empfängers auszuführen.

2. Implantat nach Anspruch 1, wobei
der Durchmesser des Rings (2) größer ist als der Durchmesser der Scheibe (1).

3. Implantat nach Anspruch 1, wobei
der Durchmesser des Rings (2) zwischen 7,5 mm und 9,0 mm liegt.

4. Implantat nach Anspruch 1, wobei
der Durchmesser des Rings (2) zwischen 8,0 mm und 8,5 mm liegt.

5. Implantat nach einem der Ansprüche 1 bis 4, wobei
das radiale Verbindungselement mindestens eine Rippe (3) ist, die das ebene Element (1) und den Ring (2) in der oberen Ebene miteinander verbindet.

6. Implantat nach Anspruch 5, wobei
die mindestens eine Rippe vier Rippen (3) sind, die in azimutaler Richtung um einen Winkel von 90° voneinander beabstandet sind.

## Revendications

1. Implant comprenant :
un élément planaire (1) au centre de l'implant, un anneau (2) dans un plan supérieur parallèle à l'élément planaire, et un élément de connexion radial (3) entre l'élément planaire et l'anneau, dans lequel l'élément planaire est un disque (1), dans lequel l'implant est configuré pour réaliser la fonction d'une barrière mécanique entre la cornée du donneur et l'endothélium de Descemet hôte.

2. Implant selon la revendication 1, dans lequel le diamètre de l'anneau (2) est supérieur au diamètre du disque (1).

3. Implant selon la revendication 1, dans lequel le diamètre de l'anneau (2) est compris entre 7,5 mm et 9,0 mm.

4. Implant selon la revendication 1, dans lequel le diamètre de l'anneau (2) est compris entre 8,0 mm et 8,5 mm.

5. Implant selon l'une quelconque des revendications 1 à 4, dans lequel l'élément de connexion radial est au moins une nervure (3) interconnectant l'élément planaire (1) et l'anneau (2) dans le plan supérieur.

6. Implant selon la revendication 5, dans lequel l'au moins une nervure est quatre nervures (3) séparées les unes des autres dans la direction azimutale par un angle de 90°.
